# EUROPEAN PATENT APPLICATION

(11) **EP 4 529 943 A1**
(43) Date of publication of application: **02.04.2025**
(21) Application number: 23200756.7
(22) Date of filing: 29.09.2023
(51) Int. Cl.: A61M 37/00, A61B 5/15

(54) **NEEDLE ASSEMBLY WITH INTEGRATED GUARD**

(71) Applicant: Latch Medical Limited, 4 Dublin (IE)
(72) Inventor: Bertollo, Nicky, Dublin, 24 (IE)
(74) Representative: FRKelly

(57) **Abstract**

The present invention provides a needle assembly (10, 110) with an integrated needle guard (20, 120) comprising a first section (14, 114) and a second section (16, 116) displaceable relative to one another, at least one microneedle (18, 118) provided on each section, the first and second sections being displaceable relative to one another in order to transition the microneedles between a disengaged state and an engaged state; and a needle guard (20, 120) to automatically occlude microneedles of the assembly following use.

## Description

### Field of the invention

This invention relates to a needle assembly with an integrated needle guard, and in particular to a needle assembly employing opposed arrays of microneedles which are displaceable relative to one another between undeployed and deployed states, and which needle guard is operable to automatically cover and/or expose the microneedles during operation of the needle assembly.

### Background of the invention

Microneedles are gaining increased use in various medical applications in view of the many documented benefits for both patients and medical professionals, for example reduced tissue trauma, surgery times and patient recovery, reduced risk of infection, and minimising the surgical or medical equipment needed in procedures involving microneedle based devices. Such microneedles allow for extremely accurate, and shallow, deployment into tissue, which can be beneficial in various applications, for example in drug delivery, and more particularly in drug delivery to sensitive or delicate tissue such as skin, ocular tissue and oral mucosa.

International patent applications WO2018/069543, WO2019/201903 and WO2022/238411 provide detailed disclosures of the configuration and operation of microneedles and opposing microneedle arrays which may be provided in the form of a device for application to a tissue substrate for various surgical and therapeutic uses, one particular use being drug delivery directly from or through the microneedles provided. The disclosures of WO2018/069543, WO2019/201903 and WO2022/238411 are incorporated herein in their entirety.

While the microneedles utilised in the above devices are relatively small in dimension, in particular in length, relative to a conventional needle and thus pose a significantly lower risk of accidental needle pricks, there is still a desire to provide some form of protective cover or guard over the needles in order to protect the end user or medical professional from unintended contact with the needles following use, in particular as such needles may be coated with or contain a drug or other medicament and/or may be carrying biological matter.

It is therefore an object of the present invention to provide a needle assembly which is operable to quickly and easily deploy microneedles into a tissue substrate such as the skin and which incorporates an integrated needle guard which automatically occludes the microneedle subsequent to deployment and following removal from the target tissue.

### Summary of the invention

According to the present invention there is provided a needle assembly comprising a first section and a second section displaceable relative to one another, at least one microneedle provided on each section, the first and second sections being displaceable relative to one another in order to transition the microneedles between a disengaged state and an engaged state; and a needle guard translatable from a retracted position exposing the needles to an extended position occluding the needles in response to the relative displacement of the first and second sections.

Preferably, the first and second section are displaceable relative to one another along a first plane and the needle guard is translatable from the retracted to the extended position along a second plane substantially perpendicular to the first plane.

Preferably, the needle assembly comprises at least one coupling operable to convert the relative motion between the first and second section into the translation of the needle guard from the retracted to the extended position.

Preferably, the coupling is adapted to retain the needle guard in the retracted position as the first and second section are displaced in a first direction to transition the microneedles from an initial disengaged state into an engaged state anchored in tissue and to translate the needle guard into the extended position as the first and second section are displaced in a second direction opposite the first direction in order to transition the microneedles from the engaged state back into a disengaged stage to be released from the tissue.

Preferably, the coupling comprises a follower fixed to the first section and a guideway in which the follower is captured for movement along a path defined by the guideway.

Preferably, the path comprises a first leg along which the follower is constrained for movement when the first and second section are displaced in the first direction and a second leg along which the follower is constrained for movement when the first and second section are displaced in the second direction.

Preferably, the first leg constrains the follower for moment in the first plane and the second leg constrains the follower for movement in a direction inclined to the first plane.

Preferably, the coupling comprises a unidirectional gate adjacent an end of the second leg through which the follower can pass but not return.

Preferably, the gate comprises a resiliently deformable member located at least partially occluding the second path and adapted to be displaced out of the second path by passage of the follower in one direction only.

Preferably, the needle guard is rotatably translatable between the retracted and extended positons, the needle guard comprising a hinge at one end and the coupling is located at an opposed end.

Preferably, the needle guard comprises a wall member extending between the hinge and the coupling, the wall member increasing in dimension in the second plane between the hinge and the coupling.

Preferably, the needle assembly comprises a first needle guard in operative association with the second section in order to expose and occlude the at least one microneedle thereon and a second needle guard in operative association with the first section in order to expose and occlude the at least one microneedle thereon.

Preferably, the needle assembly comprises a release mechanism defined by a trigger on each of the first and second sections and arranged to facilitate manual displacement of the needle assembly into the disengaged state.

Preferably, the first and second section together define an enclosure at least partially surrounding a manifold adapted for a fluid tight connection with a fluid reservoir, at least one of the microneedles being hollow and in fluid communication with the manifold.

Preferably, the needle guard is captured between the first and second sections and is linearly displaceable between the retracted and the extended position.

Preferably, the needle guard is formed integrally with the first and/or the second section.

### Brief description of the drawings

The present invention will now be described with reference to the accompanying drawings, in which:
Figure 1 illustrates a side elevation of a needle assembly according to a first embodiment of the present invention in a disengaged state and with a needle guard in a raised or retracted position;
Figure 2 illustrates a side elevation of the needle assembly of Figure 1 in an engaged state with the needle guard remaining in the retracted position;
Figure 3 illustrates a side elevation of the needle assembly of Figures 1 and 2 having been returned to a disengaged state following use and with the needle guard in a lowered or extended position;
Figure 4 illustrates a perspective view of the needle assembly as shown in Figure 1;
Figure 5 illustrates a cut away perspective view of the needle assembly as shown in Figures 1 and 4:
Figure 6 illustrates a cut away perspective view of the needle assembly as shown in Figure 2:
Figure 7 illustrates a cut away perspective view of the needle assembly as shown in Figure 3:
Figure 8 illustrates a perspective view of a pair of needle guards in isolation but which in use form part of the needle assembly shown in Figures 1 to 6, the needle guard being in a retracted position corresponding to the arrangement of the needle assembly as shown in Figures 1, 4 and 5;
Figure 9 illustrates the pair of needle guards of Figure 8 still in a retracted position but corresponding to the arrangement of Figures 2 and 6 of the needle assembly,
Figure 10 illustrates the pair of needle guards of Figure 8 still in an extended position corresponding to the arrangement of the needle assembly as shown in Figures 3 and 7;
Figure 11 illustrates a side elevation of one of the needle guards shown in Figures 8 to 10 for illustrative purposes;
Figure 12 illustrates a side elevation of a needle assembly according to a second embodiment of the present invention in a disengaged state and with a needle guard in a raised or retracted position;
Figure 13 a side elevation of the needle assembly of Figure 12 in an engaged state with the needle guard remaining in the retracted position:
Figure 14 illustrates a side elevation of the needle assembly of Figures 12 and 13 having been returned to a disengaged state following use and with the needle guard in a lowered or extended position:
Figure 15 illustrates a perspective view from an underside thereof of the needle assembly as shown in Figure 14:
Figure 16 illustrates a side elevation of a needle guard in isolation but which in use forms part of the needle assembly shown in Figures 12 to 15, the needle guard being in a retracted position corresponding to the arrangement of the needle assembly as shown in Figure 12;
Figure 17 illustrates the needle guard of Figure 16 still in a retracted position but corresponding to the arrangement of Figure 13 of the needle assembly:
Figure 18 illustrates the needle guard of Figure 16 in an extended position corresponding to the arrangement of the needle assembly as shown in Figure 14: and
Figure 19 illustrates an end elevation of the needle guard illustrated in Figures 16 to 18:

### Detailed description of the drawings

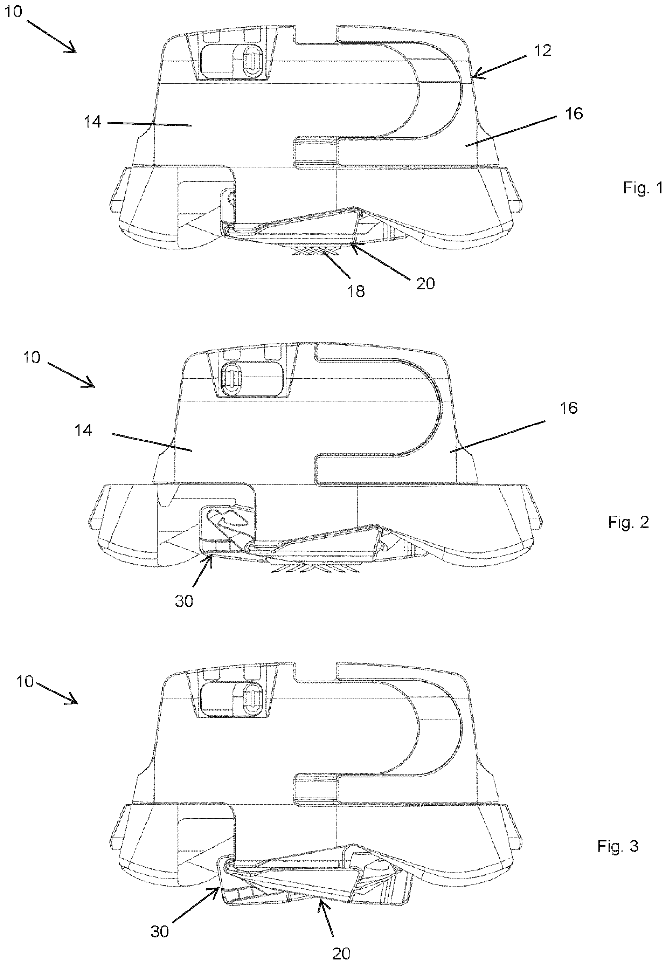

Referring now to Figures 1 to 11 of the accompanying drawings there is illustrated a first embodiment of a needle assembly, generally indicated as 10, which in the embodiment illustrated is intended for use in delivering one or more fluids, in particular a dose of a drug or therapeutic agent in liquid form, to a target area of tissue. The delivery system 10 is suitable for use with a wide range of tissue, for example skin, muscle, or ocular tissue. The needle assembly 10 is also preferably adapted to be coupled to an external fluid supply, most preferably a conventional syringe (not shown) as will be described hereinafter, to allow a fluid to be dispensed from the syringe or other external fluid supply to the target tissue. Such an arrangement is for example described and shown in the Applicant's international patent application WO2022/238411. However it is also envisaged that the needle assembly 10 may be configured for other applications, for example those disclosed in international patent applications WO2018/069543 and WO2019/201903. For the purposes of the present invention the specific application is not essential to the operation of the invention, which relates to the use of an integrated needle guard as hereinafter described.

The needle assembly 10 comprises a body 12 having a first section 14 and a second section 16 which are each provided with an array of hollow microneedles 18 extending from an underside of the respective section 14, 16 and inclined towards one another as described in detail in the above mentioned international patent applications. The hollow microneedles 18 are adapted to be reversibly insertable into the target tissue to allow for drug delivery through the hollow microneedles 18, the operation of which is described in detail in particular in WO2022/238411. As such a detailed explanation of the broad operation of the needle assembly 10 as it related to the displacement of the first and second sections 14, 16 is not considered necessary.

The number of microneedles 18 may be varied, for example to suit a particular application, target tissue area, drug to be delivered and/or delivery rate, and it is also envisaged that one or more solid microneedles (not shown) may be provided to securely anchor the hollow microneedles 18 to the target tissue to achieve reliable drug delivery. The material, dimensions, orientation, and relative positioning of the microneedles 18 may also be varied as necessary. For example the dimension and/or orientation of the microneedles 18 may be arranged to provide a desired depth of insertion into the target tissue, which can therefore facilitate accurate drug delivery to specific locations or layers of tissue.

In a preferred embodiment the body 12 is substantially formed from one or more polymers and the needle assembly 10 is intended as a single use product. The microneedles 18 may also be formed from a polymer, or may be metal or another material and suitably secured to the first and second section 14, 16. However it is also envisaged that the assembly 10 could be substantially formed from a metal such as stainless steel, titanium, etc. or a hard wearing polymer and may be reusable following sterilisation, for example in an autoclave. The microneedles 18 could be provided as a modular component releasably securable to the body 12 and thus single use while the body 12 is reusable.

The first and second sections 14, 16 of the body 12 are secured together but displaceable relative to one another by a fixed distance, and in the embodiment illustrated are slidably displaceable relative to one another to transition the needle assembly 10, and in particular the microneedles 18 between a disengaged state, for example as illustrated in Figures 1, 4 and 8, and an engaged state, for example as illustrated in Figures 2, 5 and 9. In the disengaged stage the opposed microneedles 18 are in a first position relative to one another prior to application to the target tissue, and in the engaged state are in a second position relative to one another. In use the microneedles 18 are applied against the target tissue in the disengaged state as shown in Figure 1 and the needle assembly 10 is then displaced into the engaged state as shown in Figure 2 by manually advancing the first and second section 14, 16 towards one another, which action effects the relative displacement of the opposed arrays of microneedles 18 in a manner which draws and anchors the microneedles 18 into the target tissue to allow drug delivery or other action. Once the needle assembly 10 has completed the necessary function, whether drug delivery, wound closure, biosensing (e.g. interstitial fluid extraction) or otherwise, the first and second sections 14, 16 are displaced back into the disengaged state in order to extract the microneedles 18 from the tissue and allow the needle assembly 10 to be removed from the target tissue. Again this mode of operation is described in detail in WO2018/069543, WO2019/201903 and WO2022/238411 and as a result a detailed explanation of the deployment and subsequent removal related operation of the needle assembly 10 is not considered necessary hereinafter.

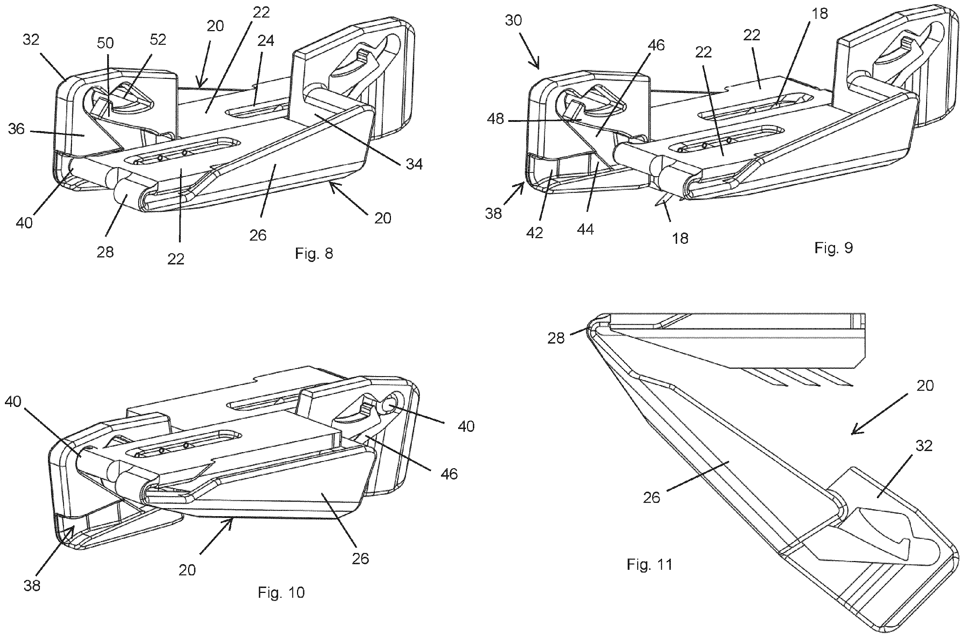

The needle assembly 10 further comprises a pair of needle guards 20, one in operative associate with each of the first section 14 and the second section 16, and which are each adapted to be automatically deployed from a raised or retracted position as shown in Figures 1, 2, 8 and 9 to a lowered or extended position as illustrated in Figures 3 and 10. With the needle guard 20 in the retracted position the microneedles 18 on the respective first or second section 14, 16 are exposed and can therefore be inserted into the target tissue as hereinbefore described, while in the extended or lowered position the microneedles 18 are occluded or generally inaccessible in order to provide a protective barrier to prevent unintended contact with the microneedles 18. The pair of needle guards 20 are shown in isolation in Figures 8 to 11 for ease of reference.

Each needle guard 20 comprises a planar base 22 which is preferably approximately the same size and shape as the underside of the first and second sections 14, 16 from which the microneedles 18 project, such that in use the base 22 is secured in face to face contact with the underside of the first or second section 14, 16 by any suitable means, whether adhesive, welding or otherwise. It is also envisaged that the base 22 may be formed integrally with the first and second section 14, 16 whereby the base 22 can be considered or defined as a lowermost layer or portion of the respective first or second section 14, 16. The base 22 defines a window 24 through which the microneedles 18 pass, particularly if the microneedles 18 are hollow and in fluid communication with a syringe (not shown) or the like from which a suitable drug is delivered in use. It can therefore be seen that the pair of needle guards 20 are located, in use, in side by side alignment and will move relative to one another as the first and second sections 14, 16 are displaced between the undeployed and deployed states. With the first and second sections 14, 16 in the undeployed state the pair of bases 22 are located side by side in the same plane but are partially longitudinally offset relative to one another, as shown in Figure 8. When the first and second sections 14, 16 are displaced into the deployed state the pair of bases 22 slide alongside one another in a first direction into a position in which they are substantially longitudinally overlapping as shown in Figure 9. Then when the first and second section 14, 16 are displaced back into the undeployed state the pair of bases 22 are slid alongside one another in a second direction opposite to the first direction to be back in a position longitudinally offset to one another as shown in Figure 10.

The needle guard 20 comprises a wall member in the form of a shutter 26 which is mounted to the base 22 via a hinge 28 positioned at one end of the base 22. The hinge 28 permits the shutter 26 to be rotated relative to the base 22 between a retracted position illustrated in Figures 8 and 9 and an extended position illustrated in Figure 10. This movement between the retracted and extended positions preferably effectively occurs in a second plane substantially perpendicular to the first plane along which the bases 22 slide relative to one another. For example if the needle assembly 10 is placed against the target tissue in a substantially horizontal orientation such that the first and second section are displaced horizontally relative to one another to effect deployment, although the shutter 26 rotates the overall motion is downwardly in a vertical direction.

Although not essential, in the preferred embodiment illustrated the hinge 28 is formed integrally with both the base 22 and the shutter 26 and comprises a so called "living hinge" although any other functional equivalent may be employed. This enables the entire needle guard 20 to be manufactured as a single component, as shown in Figure 11. The shutter 26 is positioned outboard of the respective first or second section 14, 16, is of approximately the same length of the base 22, and has a tapered profile increasing in height from the hinge 28 in a direction towards an opposite end of the base 22. In this outboard or offset position the shutter 26 is free to rotate between the retracted and extended positions to expose and occlude the microneedles 18. Although the shutter 26 does not actually overlie or cover the microneedles 18 when in the extended position, the shutter 26 extends below the tips of the microneedles 18 and thus effectively prevents unintended contact therewith. By employing the pair of needle guards 20 the microneedles 18 are also enclosed between the pair of opposed shutters 26, further reducing the likelihood of inadvertent contact when the needle guards 20 are extended.

The above described rotation of the shutter 26 is effected automatically in response to the relative displacement of the first and second sections 14, 16 as hereinafter described in detail. In particular the needle guard 20 is adapted to retain the shutter 26 in the retracted position both while the needle assembly 10 is initially in the undeployed state prior to application to the target tissue and also while deployed on the target tissue with the microneedles embedded therein. The needle guard 20 is further adapted to automatically displace the shutter 26 into the extended position as the needle assembly 10 is moved back into the undeployed state and removed from the target tissue. In this way the microneedles 18 will be occluded following use of the needle assembly 10 in order to prevent accidental contact therewith prior to appropriate disposal of the needle assembly 10. While the above described operation is based around rotation of the shutters 26 it will be appreciated that displacement from the retracted to the extended position could be achieved through a sliding rectilinear displacement or the like, or any other functional alternative.

In order to achieve this automated displacement the needle guard 20 comprises a coupling 30 operable to automatically translate the relative displacement between the first and second sections 14, 16 into displacement of the shutter 26 from the retracted to the extended position. As described in detail hereinafter, the coupling 30 is also preferably adapted to permit unidirectional displacement of the shutter from the retracted position to the extended position, in order to ensure that once extended the shutter 26 cannot be accidentally or intentionally returned to the retracted state in order to effectively provide a tamper proof safety feature preventing re-use. The coupling 30 includes a guide plate 32 fixed to the respective shutter 26 at the end opposite the hinge 28 via a transverse end wall 34. The guide plate 32 projects longitudinally beyond the end of the respective base 22 and defines an inner face 36 lying substantially in the second plane, parallel and contiguous with an inner edge of the adjacent base 22, such as to be able to move past and alongside the adjacent base 22 when the first and second sections 14, 16 are displaced relative to one another. Formed in relief in the inner face 36 is a guideway 38, the coupling 30 further comprising a follower 40 projecting from the adjacent needle guard 20 and which is captured for constrained motion within the guideway 38. The follower 40 is in the form of a short bar or pin which projects from the end of the base 22 opposite the hinge 28. The follower 40 may however extend from another suitable location, for example somewhere directly on the first or second section 14, 16 and need not extend from the base 22.

With the first and second sections 14, 16 in the initial undeployed state prior to application to the target tissue, for example as shown in Figure 8, the follower 40 is located at an open end of a first leg 42 of the guideway 38. The first leg 42 extends substantially parallel to the first plane and thus parallel to the direction of relative displacement between the first and second sections 14, 16. Thus as the first and second sections 14, 16 are displaced into the deployed state as shown in Figure 9, the follower 40 will be driven along the first leg 42 towards an opposite closed end thereof. However as the first leg 42 is parallel to the displacement of the first and second sections 14, 16 the relative motion between the follower 40 and guideway 38 does not generate and displacement of the guide plate 32 in the second plane. It can be seen in Figure 9 that the guide plate 32 has remained in the same position relative to the second plane. As a result the respective shutter 26, connected to the guide plate 32, has remained in the retracted position.

Referring in particular to Figure 9 it can be seen that the first leg 42 defines a tapered increase in wall thickness from the open end towards the closed end, before a stepped return to full depth at a unidirectional first gate 44 located upstream of the end of the first leg 42. This defines a ramp formed in the first half of the first leg 42 which will therefore act to deflect the guide plate 32 laterally away from the adjacent base 22 as the follower 40 advances up this ramp before passing over the step at which point the follower 40 drops over the ramp and is again fully accommodated in the first leg 42 allowing the guide plate 32 to deflect back into position against the adjacent base 22. However the follower 40 will not be able to return along the first leg 42 towards the open end therefore due to the impediment of the gate 44 defined by the stepped face of the ramp. The necessary degree of deflection may for example be achieved through material selection and/or mechanical displacement of the guide plate 32.

When the needle assembly 10 is ready to be removed from the target tissue, referring to Figures 9 and 10, the first and second sections 14, 16 are displaced back into the undeployed state by sliding the sections 14, 16 relative to one another in a direction opposite to that used during deployment. This action will draw the microneedles 18 out of the target tissue to allow the needle assembly 10 to be removed and discarded. This motion will also simultaneously and automatically act to drive the follower 40 back along the first leg 42, but as noted above the follower 40 cannot move passed the first gate 44. The guideway 38 however defines a second leg 46 which is inclined upwardly away from the closed end of the first leg 42, and the stepped face of the first gate 44 is provided with a correspondingly inclined face most clearly seen in Figure 11. In Figure 11 the shutter 26 and guide plate 32 have been artificially lowered away from the microneedles 18 and base 22 in order to more clearly illustrate the construction thereof.

As a result of the provision of the unidirectional first gate 44, during this reverse displacement of the first and second sections 14, 16 the follower 40 will be driven up the second leg 46 and as the follower 40 is fixed to the base 22 this will effect displacement of the guide plate 32 relative to the adjacent base 22, thereby resulting in displacement of the shutter 26 into the extended position as shown in Figure 10. In order to prevent the shutter 26 from being moved back out of the extended position the guideway 38 comprises a second gate 48 formed adjacent an upper end of the second leg 46 and defined by a resiliently deformable arm 50. This arm 50 is profiled to be deformed into a cavity 52 formed in the guide plate 32 in response to the passage of the follow 40, and will then spring back into position blocking the path back down the second leg 46, thus permitting unidirectional passage of the follower 40. As a result the shutter 26, once displaced into the extended positon, cannot be retracted for safety reasons. It will be appreciated that the configuration and operation of both the first gate 44 and the second gate 48 may be varied once the above described functionality is achieved, namely the unidirectional passage through the gates 44, 48.

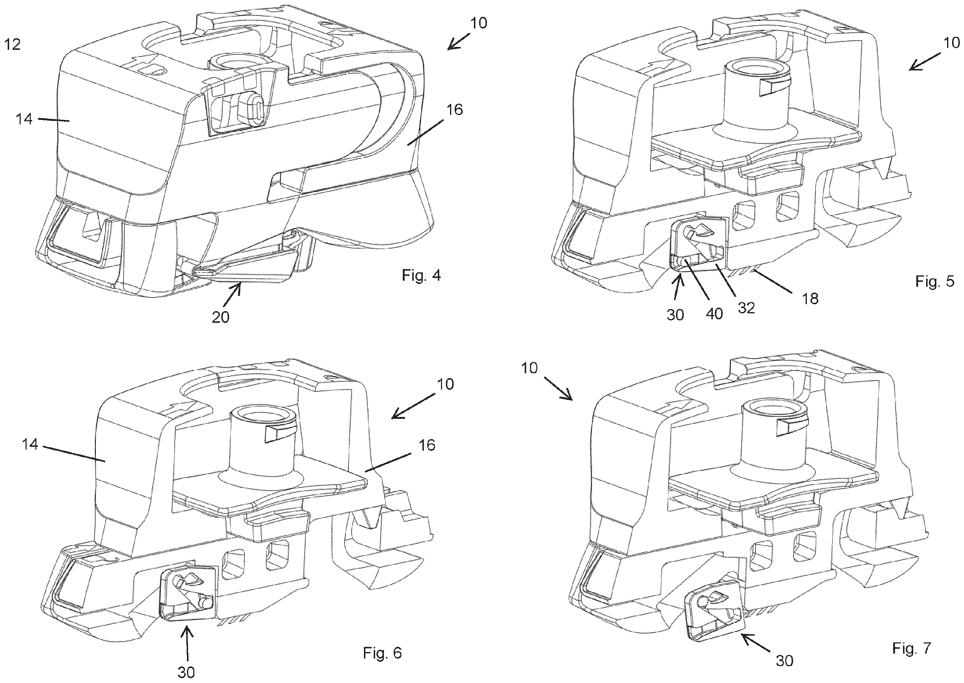

Figures 1 to 3 show the full needle assembly 10 as it is moved through the three distinct phases with the operation of the needle guards 20, including the respective coupling 30, being visible about the underside of the assembly 10, while Figures 5 to 7 show the same stages but in a cutaway view. Again it is to be understood that while the needle assembly 10 is of a particular configuration for use with a syringe (not shown) in order to deliver a drug or other medicament through the hollow microneedles 18 it will be understood that this is not essential for the operation of the automated needle guards 20, which may be used with any other suitable configuration of needle assembly that employs opposed pairs of microneedles displaceable relative to one another to effect deployment.

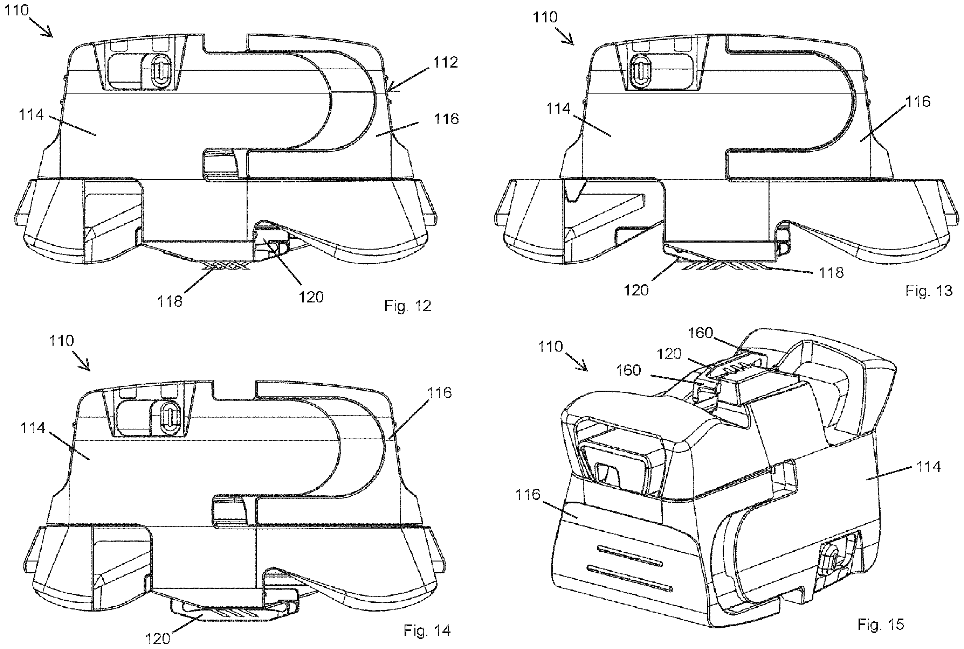

Referring now to Figures 12 to 19 there is illustrated a second embodiment of a needle assembly according to the present invention, generally indicated as 110. In this second embodiment like components have been accorded like reference numerals and unless otherwise stated perform a like function.

As with the first embodiment the needle assembly 110 is intended for use in delivering one or more fluids, in particular a dose of a drug or therapeutic agent in liquid form, to a target area of tissue. The delivery system 110 is suitable for use with a wide range of tissue, for example skin, muscle, or ocular tissue. The needle assembly 110 is also preferably adapted to be coupled to an external fluid supply, most preferably a conventional syringe (not shown) as will be described hereinafter, to allow a fluid to be dispensed from the syringe or other external fluid supply to the target tissue. The needle assembly 110 comprises a body 112 having a first section 114 and a second section 116 which are each provided with an array of hollow microneedles 118 extending from an underside of the respective section 114, 116 and inclined towards one another as described above in relation to the first embodiment. The hollow microneedles 118 are therefore also adapted to be reversibly insertable into the target tissue to allow for drug delivery through the hollow microneedles 118.

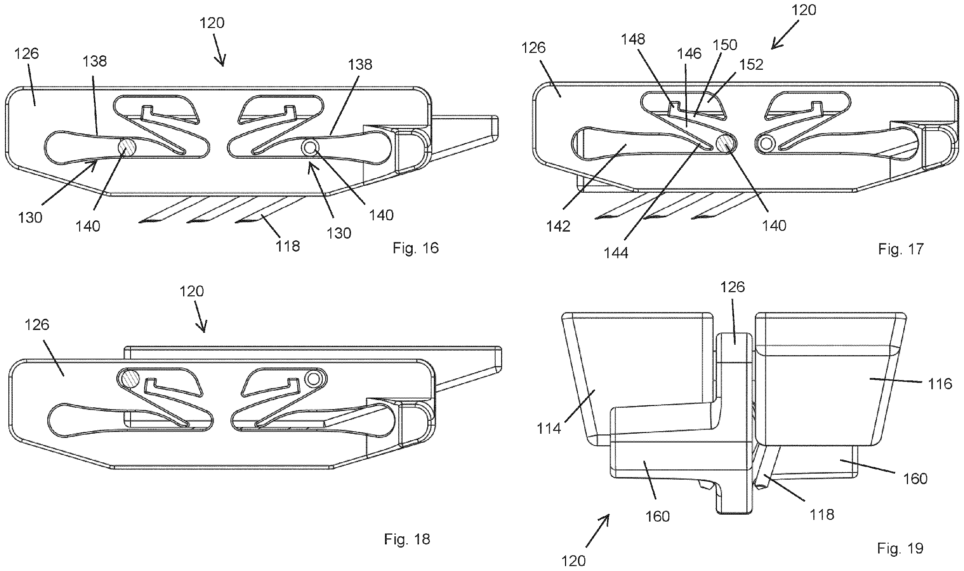

The first and second sections 114, 116 of the body 112 are again secured together but displaceable relative to one another by a fixed distance, and in the embodiment illustrated are also slidably displaceable relative to one another to transition the needle assembly 110, and in particular the microneedles 118 between a disengaged state, for example as illustrated in Figures 12 and 16, and an engaged state, for example as illustrated in Figures 13 and 17 in the same manner as hereinbefore described with respect to the first embodiment.

The needle assembly 110 further comprises a needle guard 120 in operative associate with both the first section 114 and the second section 116 by virtue of being captured therebetween but displaceable relative thereto, and which is adapted to be automatically deployed from a raised or retracted position as shown in Figures 12, 13, 16 and 17 to a lowered or extended position as illustrated in Figures 14, 15, 18 and 19. In particular the needle guard 120 comprises a substantially planar shutter 126 sandwiched between the first and second sections 114, 116. With the needle guard 120 in the retracted position a lower edge of the shutter 126 is raised above the level of the microneedles 118 and thus forms no impediment to their anchoring into tissue. The microneedles 118 on the first and second sections 114, 116 are exposed and can therefore be inserted into the target tissue as hereinbefore described, while with the shutter 126 in the extended or lowered position the microneedles 118 are occluded or generally inaccessible in order to provide a protective barrier to prevent unintended contact with the microneedles 118. The needle guard 120 is shown in isolation in Figures 16 to 19 for ease of reference.

In order to achieve this automated displacement the needle guard 120 comprises a pair of couplings 130, one in operative association with each of the sections 114, 116 and together operable to automatically translate the relative displacement between the first and second sections 114, 116 into linear displacement of the shutter 126 from the retracted to the extended position. As described with respect to the first embodiment, the couplings 130 are also adapted to permit unidirectional displacement of the shutter 126 from the retracted position to the extended position, in order to ensure that once extended the shutter 126 cannot be accidentally or intentionally returned to the retracted state. Each coupling 130 comprises a guideway 138 formed in relief or extending completely through the wall of the shutter 126. Each of the couplings 130 further comprising a follower 140 projecting from the adjacent first or second section 114, 116 and which is captured for constrained motion within the respective guideway 138. The follower 140 is in the form of a short bar or pin which projects from a sidewall of the respective first or second section 114, 116 that is in abutting contact with the face of the shutter 126.

With the first and second sections 114, 116 in the initial undeployed state prior to application to the target tissue as shown in Figures 12 and 16, each follower 140 is located at one end of a first leg 142 of the guideway 138. The first leg 142 extends substantially parallel to the first plane and thus parallel to the direction of relative displacement between the first and second sections 114, 116. Thus as the first and second sections 114, 116 are displaced into the deployed state as shown in Figure 13 and 17, the follower 140 will be driven along the first leg 142 towards an opposite end thereof. However as the first leg 142 is parallel to the displacement of the first and second sections 114, 116 the relative motion between the follower 140 and guideway 138 does not generate any displacement of the shutter 126 in the second plane. It can be seen in Figure 13 and 17 that the shutter 126 has remained in the same position relative to the second plane, namely in the retracted position.

Referring in particular to Figure 17 it can be seen that the first leg 142 tapers in height towards the second end to define a unidirectional first gate 144 located upstream of the end of the first leg 142. The first gate 144 comprises a narrow and resiliently deformable tab 144 which will be deformed upwardly in response to pressure from the follower 140 being driven along the first leg 142, before deforming back into place once the follower 140 has passed, as shown in Figure 17. The geometry of the tab 144 prevents such resilient deformation in the opposite direction, and thus defines a unidirectional gate through which the follower 140 can not return.

When the needle assembly 110 is ready to be removed from the target tissue, referring to Figures 17 and 18, the first and second sections 114, 116 are displaced back into the undeployed state by sliding the sections 114, 116 relative to one another in a direction opposite to that used during deployment. This action will draw the microneedles 118 out of the target tissue to allow the needle assembly 110 to be removed and discarded. This motion will also simultaneously and automatically act to drive each follower 140 back along the respective first leg 142, but as noted above the follower 140 cannot move past the first gate 144. The guideway 138 however defines a second leg 146 which is inclined upwardly away from the second end of the first leg 142, and the back side of the first gate 144 defines an inclined face up which the follower 140 will therefore be driven and as a result, during this reverse displacement of the first and second sections 114, 116, the follower 140 will be driven up the second leg 146 and as the follower 140 is fixed to the first or second section 114, 116 this will effect displacement of the shutter 126 into the extended position as shown in Figure 18. Unlike the first embodiment the displacement of the shutter 126 is not rotatory around a hinge but is a linear displacement which is achieved by having the two spaced apart couplings 130 formed in the shutter 126 and which therefore simultaneously effects displacement of the shutter 126 at two discrete locations in order to ensure that the shutter 126 remains stable and maintains a level attitude as it undergoes the linear displacement into the extended position.

In order to prevent the shutter 126 from being moved back out of the extended position the guideway 138 comprises a second gate 148 formed adjacent an upper end of the second leg 146 and defined by a resiliently deformable arm 150. This arm 150 is profiled to be deformed into a cavity 152 formed at the upper end of the guideway 130 in response to the passage of the follow 140, and will then spring back into position blocking the path back down the second leg 146, thus permitting unidirectional passage of the follower 140. As a result the shutter 126, once displaced into the extended positon, cannot be retracted for safety reasons. It will be appreciated that the configuration and operation of both the first gate 144 and the second gate 148 may be varied once the above described functionality is achieved, namely the unidirectional passage through the gates 144, 148.

To further avoid accidental contact with the tips of the microneedles 118 the needle guard 120 is preferably provided with a lateral buffer 160 at each end thereof and which projects transversely from a respective sidewall of the shutter 126, as most clearly visible in Figures 15 and 19. The pair of buffers 160 provide additional shielding around the tips of the microneedles 118 in order to avoid contact therewith. It will be appreciated that the particular location, size and orientation of the buffers 160 may be varied while retaining the underlying functionality thereof.

It is also envisaged that in a modified embodiment, stored potential energy in a member (not shown) is released when the follower 40; 140 is within the second leg 46; 146 such that the shutter 26; 126 immediately presses and bears against the skin surface. As the first and second sections 14; 114 and 16; 116 are displaced into the undeployed state the shutter(s) 26; 126 are also displaced into the extended position with the assistance of the stored potential energy.

The needle assembly 10; 110 of the present invention therefore provides an integrated and automated means of occluding the microneedles 18; 118 once the needle assembly 10; 110 is removed from the target tissue following use, thus providing a robust and reliable safety feature,

The invention is not limited to the embodiment described herein but can be amended or modified without departing from the scope of the present invention.

## Claims

1. A needle assembly comprising a first section and a second section displaceable relative to one another, at least one microneedle provided on each section, the first and second sections being displaceable relative to one another in order to transition the microneedles between a disengaged state and an engaged state; and a needle guard translatable from a retracted position exposing the needles to an extended position occluding the needles in response to the relative displacement of the first and second sections.

2. The needle assembly of claim 1 in which the first and second section are displaceable relative to one another along a first plane and the needle guard is translatable from the retracted to the extended position along a second plane substantially perpendicular to the first plane.

3. The needle assembly of claim 1 or 2 comprising at least one coupling operable to convert the relative motion between the first and second section into the translation of the needle guard from the retracted to the extended position.

4. The needle assembly of claim 3 in which the coupling is adapted to retain the needle guard in the retracted position as the first and second section are displaced in a first direction to transition the microneedles from an initial disengaged state into an engaged state anchored in tissue and to translate the needle guard into the extended position as the first and second section are displaced in a second direction opposite the first direction in order to transition the microneedles from the engaged state back into a disengaged stage to be released from the tissue.

5. The needle assembly of claim 4 in which the coupling comprises a follower fixed to the first section and a guideway in which the follower is captured for movement along a path defined by the guideway.

6. The needle assembly of claim 5 in which the path comprises a first leg along which the follower is constrained for movement when the first and second section are displaced in the first direction and a second leg along which the follower is constrained for movement when the first and second section are displaced in the second direction.

7. The needle assembly of claim 6 in which the first leg constrains the follower for moment in the first plane and the second leg constrains the follower for movement in a direction inclined to the first plane.

8. The needle assembly of claim 6 or 7 in which the coupling comprise a unidirectional gate adjacent an end of the second leg through which the follower can pass but not return.

9. The needle assembly of claim 8 in which the gate comprises a resiliently deformable member located at least partially occluding the second path and adapted to be displaced out of the second path by passage of the follower in one direction only.

10. The needle assembly of any of claims 3 to 9 in which the needle guard is rotatably translatable between the retracted and extended positons, the needle guard comprising a hinge at one end and the coupling is located at an opposed end.

11. The needle assembly of claim 10 in which the needle guard comprises a wall member extending between the hinge and the coupling, the wall member increasing in dimension in the second plane between the hinge and the coupling.

12. The needle assembly of any preceding claim comprising a first needle guard in operative association with the second section in order to expose and occlude the at least one microneedle thereon and a second needle guard in operative association with the first section in order to expose and occlude the at least one microneedle thereon.

13. The needle assembly of any preceding claim comprising a release mechanism defined by a trigger on each of the first and second sections and arranged to facilitate manual displacement of the needle assembly into the disengaged state.

14. The needle assembly of any of claims 3 to 9 in which the needle guard is captured between the first and second sections and is linearly displaceable between the retracted and the extended position.

15. The needle assembly of any preceding claim in which the needle guard is formed integrally with the first and/or the second section.
